# EUROPEAN PATENT SPECIFICATION

(11) **EP 1 664 770 B1**
(45) Date of publication and mention of the grant of the patent: **14.11.2007**
(21) Application number: 04774923.9
(22) Date of filing: 03.09.2004
(51) Int. Cl.: G01N 33/50

(54) **METHOD FOR DETERMINING THE IMPACT OF A MULTICOMPONENT SYNTHETIC PRODUCT MIXTURE ON THE BIOLOGICAL PROFILE OF A DISEASE WITHIN A GROUP OF LIVING SYSTEMS AND THE DEVELOPMENT OF NEW COMBINATORIAL INTERVENTIONS**
VERFAHREN ZUR BESTIMMUNG DER AUSWIRKUNG EINES AUS MEHREREN KOMPONENTEN BESTEHENDEN SYNTHETISCHEN PRODUKTGEMISCHS AUF DAS BIOLOGISCHE PROFIL EINER KRANKHEIT IN EINER GRUPPE LEBENDER SYSTEME SOWIE ENTWICKLUNG NEUER KOMBINATORISCHER INTERVENTIONEN
PROCEDE POUR DETERMINER L'IMPACT D'UN MELANGE DE PRODUITS SYNTHETIQUES A PLUSIEURS COMPOSANTS SUR LE PROFIL BIOLOGIQUE D'UNE MALADIE AU SEIN D'UN GROUPE DE SYSTEMES VIVANTS ET POUR LE DEVELOPPEMENT DE NOUVELLES INTERVENTIONS COMBINATOIRES

(30) Priority: 05.09.2003 EP 03077804
(43) Date of publication of application: 07.06.2006
(73) Proprietor: Nederlandse Organisatie voor Toegepast-Natuurwetenschappelijk Onderzoek TNO, 2628 VK Delft (NL)
(72) Inventor: WANG, Mei, NL-2341 BV Oegstgeest (NL); WITKAMP, Renger, NL-3662 LM Wijk bij Duurstede (NL); VAN DER GREEF, Jan, NL-3971 BM Driebergen (NL)
(74) Representative: van Loon, C.J.J.
(86) International application number: PCT/NL2004/000617
(87) International publication number: WO 2005/024421

(56) References cited:
- WO-A-97/20076
- WO-A-98/57174
- WO-A-03/017177
- US-A1- 2003 044 846
- US-A1- 2003 096 309
- WANG ET AL PHYTOTHERAPY RESEARCH vol. 19, no. 3, March 2005, pages 173 - 182

## Description

The present invention is related to the field of life sciences and the domain of health and diseases, in particular the development of strategies and synthetic products for the prevention, treatment or curing of a disease.

In contrast with the reductionistic approach as conventionally applied by pharmaceutical industry to develop new medicaments, the present invention is based on a holistic approach to living organisms and subsequent multicomponent intervention strategy.

A holistic approach to living organisms has, for instance, been the basis of medicine in ancient cultures especially using herbal derived products. An important starting point in for instance herbal medicine based approaches is that every healthy organism is in balance. Balance is considered to be a complex interplay between body and mind, which is reflected at all levels ranging from the biochemical component perspective to the energetic system control of our physical body. Internal imbalances can stem from a wide variety of factors and lead to a plethora of conditions ranging from short perturbations to chronic disease processes. Additionally, the uniqueness of each human being is recognized and drives the necessity to develop a personalized medication to obtain optimal results based on multi-component treatments.

At a first glance the modern western scientific medical approach may seem very different from this. However, the revolution in genomics that has taken place in life sciences during the last decade has provided considerable support for a more holistic view on diagnosis and treatment. Furthermore, the issue of personalized medicine is now receiving considerable attention due to the new insights in i.a. pharmacogenomics. Although the principle of homeostasis has been a cornerstone of Western physiology for more than a century, the enormous complexity of biological systems has often driven pharmaceutical research towards trying to identify and influence only single targets that make the difference between health and disease. This approach has indeed yielded many potent drugs but also revealed major drawbacks. In fact one tries to influence a system by interacting with a single protein that is often part of a complex pathway and involved in a cascade of reactions and feedback loops. The reality is that most diseases are multi-factorial which means that treating a single target provides a partial treatment (reduction of symptoms) and in the majority of cases no cure. Although this awareness is not new, it has been impossible to find alternative routes giving the mentioned complexity of the system.

A method has, however, now been developed that enables highly detailed profiling and subsequent measurements of multicomponent induced changes in *in-vitro* systems (such as cell cultures). In this method the interaction of multiple components with living biological systems can very effectively be measured, using a particular set of steps wherein technologies are applied such as biostatistics and bioinformatics. By means of such measurements the impact of multicomponent mixtures on the biological profile of a disease can advantageously be determined. Moreover, such measurements enable the choice of effective and safe components within the multicomponent mixtures and their respective concentrations required for having an impact on the biological profile of the disease can be identified. The invention is different from previous concepts such as described in US 2003/0096309 A1 (Borisy et al.) and WO 97/20076 (Schmidt et al.) and which concern the design of multicomponent mixtures for application in treating disease, because the present invention does not relate to measuring a biological outcome at a cellular level, but provides the measurement at a systems level which is crucial for almost all multifactorial diseases. For instance the early start of a disease is often characterised by a shift in balance between different organizational structures in a system and biochemical communication and control signals are typically found at a systems level not a single cell type level. Clearly at increasing levels of complexity in a system new properties are evolving. This communication and control element is found among others in bodyfluids present in a system such as blood, CSF or reflections thereof in urine. The present invention enables the discovery of system descriptors, biomarkers describing lower and higher levels of organisation and control, and uses this information to optimize mixtures to address the disregulation at different pathways and different system levels.

Accordingly, the present invention relates to a method as defined in claim 1.

In a preferred embodiment of the present invention after step (d) a step (e) is carried out in which one or more mixtures of said set are selected which selected mixtures display the desired impact on the biological profile of the disease.

In the context of the present invention, a multicomponent synthetic product mixture is defined as a mixture of synthetic compositions, which compositions have been produced by chemical synthesis, not by a natural process.

The living systems include human beings and all sorts of animals. When use is made of animals, the group of living systems is suitably selected from one type of animal.

The method according to the present invention enables the measurement of the effects of multiple target interventions and the development of products to optimally perform such interventions by a unique approach, revealing the biological profile of the effective components. This unique approach is referred to as multidimensional pharmacology (MDP) and uses the Systems Biology approach in which biological systems are studied by measuring and integrating metabolic data and other profile data, such as genetic and/or proteomic data.

The determination of biological effects and in particular synergetic multicomponent effects in accordance with the present invention is illustrated by the example of synthetic products in intervention strategies. The determination of such effects is not limited to biological and synergetic effects in mammalian systems but can address all possible forms of living systems with complex mixtures derived from the same portfolio of life.

The present invention is not limited to a particular type of disease, but embraces any disease of which a biological profile can be determined. In addition it can be used in prevention strategies.

A considerable advantage of the present invention resides in the fact that it provides a scientific basis for both efficacy and safety of the tested multicomponent mixtures and it provides a tool for the development strategy of combinatorial approaches.

A further very important aspect of the method according to the present invention is that it allows the measurement of all biological effects including additive and synergetic effects.

In health and disease studies a bodyfluid profile of for instance a plasma sample from a control group (reference group) and patient group (a group with symptoms of the disease with the biological profile) can be used to measure as many components as possible and is evaluated for differences in single components or patterns of components between the two groups to obtain a better insight in the underlying biological mechanisms, to detect novel biomarkers/surrogate markers, to predict toxicology or pharmacological response or to develop novel intervention routes. In the context of the present application a biomarker is defined as a characteristic that is objectively measured and evaluated as an indicator of: normal biological processes, pathogenic processes or pharmacological response to a therapeutic intervention. Biomarkers can be genes, transcripts, proteins, metabolites, (trace)-elements or any combination of those components.

The concept of the present invention of using patterns for dynamic modeling directly or after non-linear or linear multidimensional compression opens the unique route of studying dynamical processes using systems descriptions based on component patterns. In such studies perturbations as for instance via drug intervention routes can be monitored and evaluated in a multidimensional way and methods such as time series analysis, time warping and non-linear dynamic techniques can be applied. In the evaluation of data generated with the method of the present invention also the addition of other data and information from other sources can be of importance such as information from clinical dossiers of patients describing the medical diagnosis and clinical chemistry, or disease studies data on behavior, cognition, psychological, social, etc., levels. These data can be included or linked to the data set created by the method according to the present invention to classify patients or to discover sub-classes or other relevant observations. Especially in the design of new combinatorial interventions, animal models are often preferred to yield biological acitivity, for instance the measurement of insulin resistance in the study of metabolic syndrome (obesity, diabetes II, hypertension and other CV related diseases). The direct correlation of such data with the composition of the mixture and the biological response profile in that model is a preferred embodiment of this invention when new synthetic compositions are within the mixture (pre-clinical approach). For mixtures containing synthetic compositions already used or allowed to be used in man, direct profiling using bodyfluids form human beings or animals is preferred (clinical approach)

The application of the present invention creates a wide variety of novel approaches in using metabolomics, proteomics, transcriptomics and/or other component profiles of bodyfluids to enhance the overall process of biomedicine or drug discovery, development including clinical evaluation, diagnostic applications and post-marketing surveillance. The created profiles can be used first of all to obtain a better insight in the underlying biological process a.o. for toxicology evaluation (predictive toxicology), biomarker/surrogate marker or biomarker/surrogate marker pattern discovery, protein target validation, comparison of animal models and relating these to human studies, phenotyping at the metabolite level, responder/non-responder evaluation, validation of animal models such as transgenic models, providing the ability to correlate metabolite level data with other levels in systems biology such as gene, mRNA, RNAi and protein data.

The method according to the present invention generates patterns of components and the used multivariate analysis generates patterns of relevant components for a given situation or investigation. In modern biology and related nutraceutical, pharmaceutical and biotechnological industries this is a crucial new paradigm for driving the scientific research and industrial discovery and development processes. The basic understanding is that biology in general and disease processes in particular are multifactorial of nature and therefore the understanding of such processes requires a description or understanding based on a multitude of components. From recent studies using Systems Biology (Clish et al. Integrative biological analysis of the APOE*3 Leiden transgenic mouse, OMICS 2004, 1:3-13) the interconnectivity and interdependence of the components of the system become evident and the fact that new properties of systems are revealed as increasing levels of complexity are studied. In most cases development processes are for instance based on a single target evaluated with a single biomarker for efficacy or for differentiating of control groups from patients groups. The present invention, however, provides a method that allows the creation of a breakthrough in the ability to describe multifactorial diseases by multifactorial patterns as well as multifactorial responses toward multifactorial input variables such as in combination therapies.

In accordance with the present invention preferably at least one spectrometric technique, at least one electromigration-based technique or at least one chromatographic technique is used in step (a) to determine the profile of the disease. More preferably, use is made of two or more spectrometric techniques. However, special detection techniques such as laser-induced fluorescence and electrochemical detection in combination with separation techniques (e.g. (nano)-HPLC, electromigration-based methods) are also included. Most preferably, use is made of at least a nuclear magnetic resonance technique and a mass spectrometry technique to determine the profile of the disease in step (a).

The biological profile to be determined in step (a) includes one or more metabolic, genetic and/or proteomic profiles. Any combination of these profiles can be used. Preferably, such combination includes one or more metabolic profiles. More preferably, the biological profile includes metabolic, genetic and proteomic profiles.

In step (a) preferably the biological profile is determined of at least one type of bodyfluid, or at least one type of tissue or cell line. More preferably, in step (a) the biological profiles are determined of at least two different types of bodyfluid.

The biological profiles are determined in step (a) using one or more of the following biomarkers; genes, transcripts, proteins, metabolites and (trace) elements.

In step (b) preferably use is made of at least one spectrometric technique, at least one electromigration-based technique or at least one chromatographic technique to determine the impact of the series of samples of the multicomponent mixture on the biological profile of the disease. More preferably, use is made of two or more spectrometric techniques, whereas most preferably use is made of at least a nuclear magnetic resonance technique and/or a mass spectrometry technique. In the case of protein profiling other techniques such as gel-based electrophoretic techniques are included as well.

In step (d) preferably use is made of at least one spectrometric technique, at least one electromigration-based technique or at least one chromatographic technique to determine the impact of the set of multicomponent mixtures on the biological profile of the disease. In steps (c) and (d)), the design and optimization of the multicomponent mixture to match both the efficacy and safety profile desired for a given intervention are established.

The multicomponent mixture to be used in accordance with the present invention may be any multicomponent mixture having an (potential) impact on a biological profile of a disease.

In each of steps (a), (b) and (d) preferably use of at least one spectrometric technique. Suitably a nuclear magnetic resonance technique ("NMR") or mass spectrometry technique ("MS") can be used, whereby the latter technique focuses on a limited number of small molecule compounds. Both of these techniques have however limitations. The NMR approaches are limited in that they typically provide reliable information only of compounds present at high concentration. On the other hand, focused mass spectrometry techniques do not require high concentrations but can provide information of only limited portions of a biological profile. As used herein, the terms "small molecule" and "metabolite" are used interchangeably. Small molecules and metabolites include, but are not limited to, lipids, steroids, amino acids, organic acids, bile acids, eicosanoids, peptides, carbohydrates and trace elements.

Therefore, in each of steps (a), (b) and (d) preferably use is made of at least a nuclear magnetic resonance technique and/or a mass spectrometry technique.

Sample preparation for NMR can generally be very straightforward using freeze-drying and reconstitution in D₂O because the focus is on the higher concentration components, i.e. of typical concentration > 100 nanogram/mL. For MS a variety of sample preparation approaches can be used ranging from solid phase extraction and liquid/liquid extraction to more specific methods using, for instance, affinity-based methods or derivatization procedures both for GC-MS as well as LC-MS.

In each of steps (a), (b) and (d) use can be made of spectrometric data obtained from one or more platforms including, but not limited to, MS, NMR, liquid chromatography ("LC"), gas-chromatography ("GC"), high performance liquid chromatography ("HPLC"), capillary electrophoresis ("CE"), and any known form of hyphenated mass spectrometry in low or high resolution mode, such as LC-MS, GC-MS, CE-MS, LC-UV, MS-MS, MSⁿ, etc. Typical profile data can also be obtained by using more component specific detectors such as laser-induced fluorescence and electrochemical detection.

As used herein, the term "spectrometric data" includes data from any spectrometric or chromatographic technique. Spectrometric techniques include, but are not limited to, resonance spectroscopy, mass spectroscopy, and optical spectroscopy. Chromatographic techniques include, but are not limited to, liquid phase chromatography, gas phase chromatography, and electrophoresis.

If a spectral profile is obtained, as with standard spectroscopic methods, a primary necessary step is to adjust for minor shifts in the spectra both in the intensity dimension as well as in the spectral or chromatographic dimension. Shifts can be due to instrumental factors, environmental conditions, or to varying concentrations of components (as is often the case in urine analysis). As an example, variation in NMR chemical shifts often occurs and needs to be accounted for, but the reproducibility and standardization in the intensity (or peak area) of a single profile (quantitation dimension) is typically very satisfactory. This is in contrast to MS where the peak intensity (ion abundance) dimension needs to be carefully adjusted or standardized due to lack of calibrants for each component present in the profile. In hyphenated techniques the reproducibility of the separation method (GC, LC or electromigration driven techniques such as capillary electrophoresis (CE)) needs to be carefully evaluated as well. In this respect near-infrared spectral profiles are impressive and correction in either dimension is hardly required.
In general, small instrumental shifts in the spectral (variable) dimension will be falsely interpreted as representing different components when a collection of data profiles is subjected to pattern recognition analysis. A straightforward way to cope with this problem is by using binning techniques in which the spectrum is reduced in resolution to a sufficient degree to insure that a given peak remains its bin despite small spectral shifts between analyses. For example, in NMR the chemical shift axis may be descretized and coarsely binned, and in MS the spectral accuracies may be rounded to integer atomic mass unit values. However, more subtle procedures are preferred such as partial linear fit for NMR or other alignment procedures for MS.

After initial data pre-processing the spectral profiles are set for pattern recognition (multivariate analysis).

The ability to use different techniques to produce bodyfluid profiles is optimally used by multiway multivariate analysis and allows the measurement of different bodyfluids of the same system (such as plasma and urine or plasma and CSF) to reveal novel insights into the systems biology, for instance the effect of the blood brain barrier when comparing plasma and urine profiles. Hence, in each of steps (a)-(c) preferably use is made of multiway multivariate analysis.

The present invention provides a method of spectrometric data processing utilizing multiple steps of multivariate analysis to process data in a hierarchal procedure (steps (a), (b) and (d)). In each of steps (a), (b) and (c) a first multivariate analysis can be used on a plurality of data sets to discern one or more sets of differences and/or similarities between them, whereafter a second multivariate analysis can be used to determine a correlation (and/or anti-correlation, i.e., negative correlation) between at least one of these sets of differences (or similarities) and one or more of the plurality of data sets. In step (a) the determination of the biological profile of a disease may also be based on the correlation.

As used herein, the term "data sets" refers to the spectrometric data associated with one or more spectrometric measurements. For example, where the spectrometric technique is NMR, a data set may comprise one or more NMR spectra. Where the spectrometric technique is UV spectroscopy, a data set may comprise one or more UV emission or absorption spectra. Similarly, where the spectrometric technique is MS, a data set may comprise one or more mass spectra. Where the spectrometric technique is a chromatographic-MS technique (e.g., LC-MS, GC-MS, etc), a data set may comprise one or more mass chromatograms. Alternatively, a data set of a chromatograms or reconstructed TIC chromatograms. In addition, it should be realized that the term "data sets" includes both raw spectrometric data and data that has been pre-processed (e.g., to remove noise, baseline, detect peaks, etc.).

Moreover, as used herein, the term "data sets" may refer to substantially all or a sub-set of the spectrometric data associated with one or more spectrometric measurements. For example, the data associated with the spectrometric measurements of different sample sources (samples of groups with symptoms of the disease (experimental group samples) v. samples of reference or healthy groups (control group samples)) may be grouped into different data sets. As a result, a first data set may refer to experimental group sample measurements and a second data set may refer to control group sample measurements. In addition, data sets may refer to data grouped based on any other classification considered relevant.

The present invention also provides a method of spectrometric data processing utilizing multivariate analysis to process data at two or more hierarchal levels of correlation. In each of steps (a), (b) and (d) use can be made of a multivariate analysis on a plurality of data sets to discern correlations (and/or anti-correlations) between data sets at a first level of correlation, whereafter the multivariate analysis can be used to discern correlations (and/or anti-correlations) between data sets at a second level of correlation. In step (a) the determination of the biological profile of a biological system may also be based on the correlations discerned at one or more levels of correlation.

In accordance with the present invention the spectrometric data processing in each of steps (a), (b) and (d) can be carried out utilizing multiple steps of multivariate analysis to process data sets in a hierarchal procedure, whereby one or more of the multivariate analysis steps further comprises processing data at two or more hierarchal levels of correlation. For example, in each of steps (a), (b) and (d) a first multivariate analysis can be used on a plurality of data sets to discern one or more sets of differences and/or similarities between them; a second multivariate analysis can be used to determine a first level of correlation (and/or anti-correlation) between a first set of differences (or similarities) and one or more of the data sets; and the second multivariate analysis can be used to determine a second level of correlation (and/or anti-correlation) between the first set of differences (or similarities) and one or more of the data sets. In step (a) the determination of the biological profile of the disease can be based on the correlations discerned at one or more levels of correlation.

Suitable forms of multivariate analysis include, for example, principal component analysis ("PCA"), discriminant analysis ("DA"), PCA-DA, factor analysis, canonical correlation ("CC"), partial least squares ("PLS"), predictive linear discriminant analysis ("PLDA"), neural networks, multilevel/multiway/multiblock analysis, iterative target analysis, general procrustus analysis, support vector machines ("SVM"), parafac and pattern recognition techniques.

The use of the above-described multivariate analysis techniques for profiling only is as such known in the art. For a more detailed description reference van, for instance, be made to pending US Provisional Patent Application, Serial No. 60/312,145 (Method and System for Profiling Biological Systems).
To extract the maximum value from the data, multivariate analysis tools as outlined above may be used in concert with additional statistical and informatics strategies. Once statistically significant differences in, for instance, metabolite abundances are determined and quantified among groups of samples, the objective becomes to understand the underlying biological reasons for and the contexts of the results. A first step is to identify metabolic components observed in data spectra and revealed by multivariate analysis to be significant differences among samples. Such identification typically involves querying various databases of known metabolite component spectra and structures. A next step is to mine existing knowledge about molecular interactions through searches of public and private databases. This can go some way in explaining the associations and behaviour observed in the metabolomic profile results. However, because most of the metabolic, genomic, proteomic, and interaction databases depict biochemical events in a static state, progressively sophisticated analytical and mathematical tools are needed to integrate disjointed biological clues into dynamic models that are better suited to explain, for example, pathological processes.

Indeed, both linear and non-linear multivariate analyses may uncover statistically significant associations between biomolecular components that will not be explained through the mining of existing databases or literature.

A preferred embodiment of the method of the present invention comprises the following steps:
1. A selection is made of the relevant samples, for instance bodyfluids (plasma, urine, CSF, saliva, sebum, synovial fluid etc.)
2. A selection is made of the width of the biological profiling; transcripts, proteins, metabolites, etc.
3. A sample is prepared based on the spectrometric techniques to be used for determining the biological profile (e.g. GCMS, LCMS, CEMS, MS/MS combinations and various NMR-methodologies, gel-based electrophoretic techniques etc.)
4. The profile is determined using the spectrometric techniques, gel-based techniques, NMR-profiles and preferred MS-approaches in case of metabolomics covering lipids, steroids, bile acids, eicosanoids, (neuro)peptides, vitamins, organic acids, neurotransmitters, amino acids, carbohydrates, ionic organics, nucleosides, inorganics, xenobiotics, etc., with a preference to include peptides. Also a global MS-profile to describe in a single experiment the major high concentration components can be included which is often a good indication for the balance/homeostasis of a system in addition to the NMR-profile.
5. The data obtained is preprocessed using preferable the technique described in Dutch patent application 1016034, in combination with PCA-DA, multiblock/multiway multivariate analysis based on linear and non-linear techniques and the partial linear fit algorithm.
6. The outcome of 4 is combined with other relevant data sources such as animal model based biological data, medical history, clinical chemistry records, clinical endpoints, biomakers, surrogate markers, medical description and behavioural, social, psychological data, etc.
7. The (non-linear or linear) dynamics of dynamical diseases are studied by using one of the profiling-components or any combination of the profiling components, preferably by using a combination of non-linear compression and dynamic modeling techniques.

The concept of the present invention is suitably based on the following aspects, the profiling of complex mixtures such as body fluids by a combination of NMR and a selection of hyphenated mass spectrometric techniques (GC-,LC-,-CE-MS/MS, ICPMS), the evaluation of the combination with data preprocessing/scaling preceding multiblock/multiway multivariate analysis, the combination of instrumental datasets created with other relevant datasets, the linking of data sets arising from samples from one system but via different bodyfluid profiles and the ability to study all forms of non-linear dynamics.

In accordance with the present invention synthetic product-based medicaments can be designed.

### Example

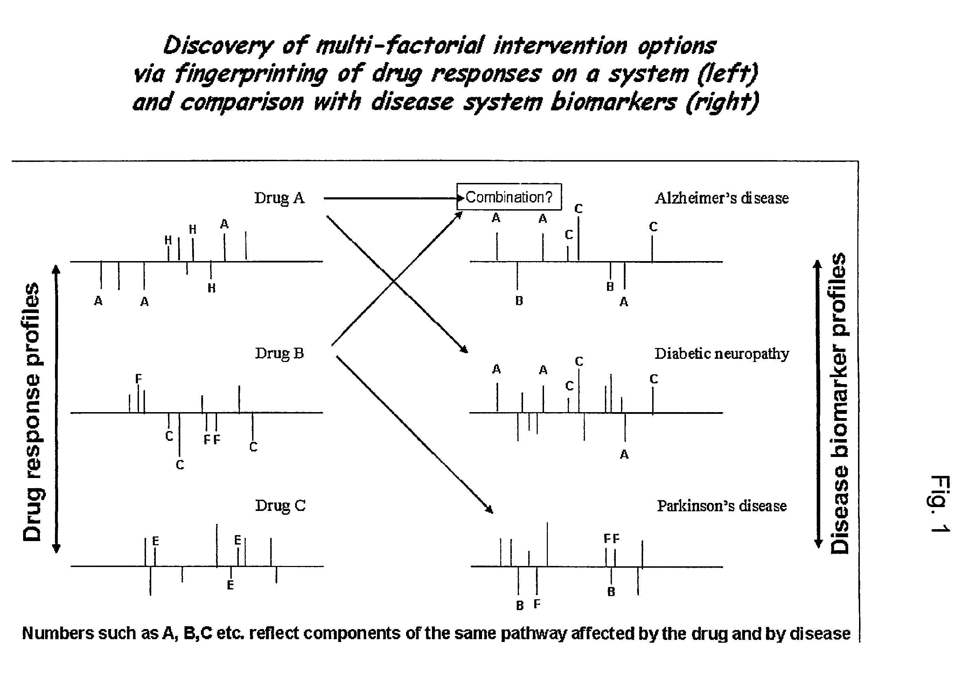

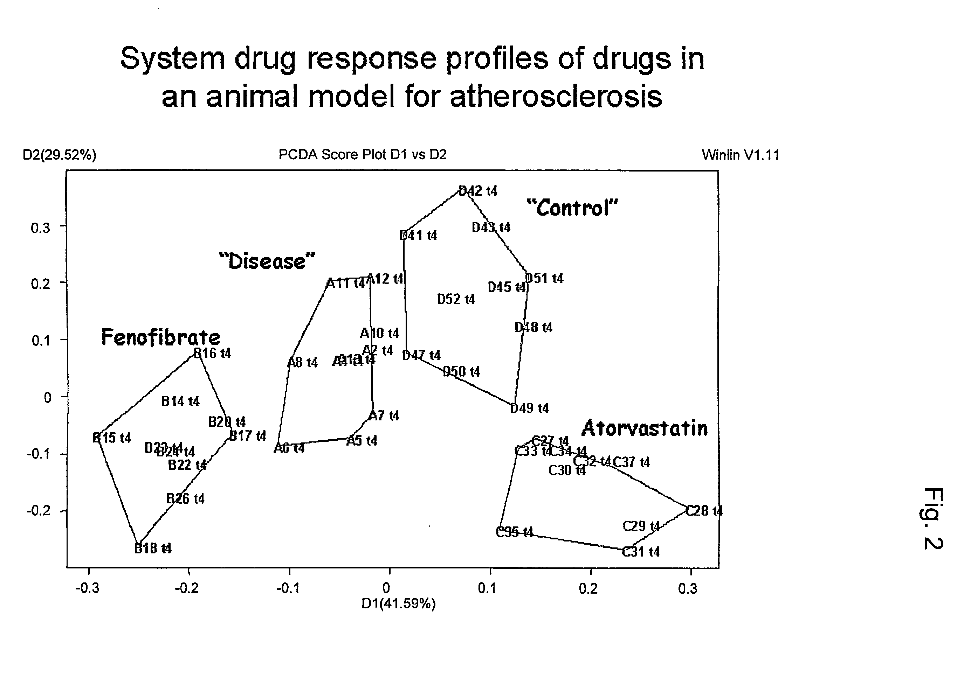

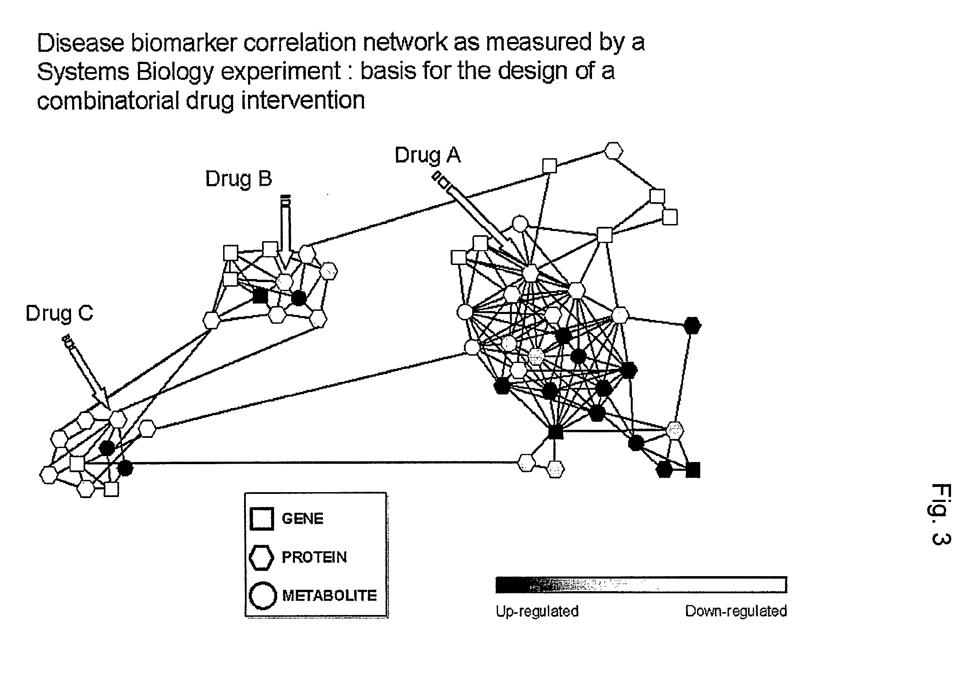

The method according to the present invention is schematically depicted in Figure 1. A typical experiment in accordance with the present invention based on the fingerprints as generated by the systems biology approach a typical experiment is based on the following steps:
1. A number single components or a set of different mixtures, preferably with a significant variation in composition, is measured (fingerprinted) by a profiling technique such as NMR or mass spectrometry; indicated as batches 1-n in the left part of Figure 1.
2. The profiles of the effect of those batches after administration into an animal model or in a human trial are measured as well as a reference group which is not treated (comprised of both disease animals and wildtype or healthy subjects/patients) or chosen to be treated in another way.
3. The reference group provides the biomarker profile for the disease and the other experiments provide the impact of the mixture on the disease pattern and reveals also other effects. In addition clinical endpoints in human trials or typical biological effects measured in cell-models and/or animal models can be used for evaluation of the multicomponent mixture. This yields a prove of effect on the specific disease group. In case a control group is not available comparative analysis can be used to reveal the optimal biological effect towards a clinical endpoint or hypothesis.
4. (Non)-linear multivariate correlation of the patterns of the mixture components and the effect profiles including also all other information such as clinical endpoints or any biological effect in other models (cell based or animal models) enables the detection of the patterns of components responsible for the biological effects.
5. In designing new combinatorial interventions the impact of single components can be studied and the impact on the disease profile determined in terms of changes in biology effected or when not identified also as impact on a certain part of the disease profile. An example of such an approach is given in Figure 2 in which the impact of different drugs on the development of atherosclerosis in an animal model is evaluated by system based responses. Clearly both drugs have different drug responses, which is only partly visualized in the 2-D representation. When analyzed in detail the differences on the impact of the disease profile can be found by correlation networks, see example Figure 3 and explained at the biochemistry level and consequently it can serve for the design of mixture strategy.

## Claims

1. An *in vitro* method for identifying within a multicomponent synthetic product mixture the components and their respective concentrations responsible for additive or synergetic effects on the biological profile of a disease within a group of living systems, which mixture is a mixture of synthetic compositions that have been produced by chemical synthesis, not by a natural process, which method comprises the steps of:
(a) determining a biological profile of the disease by comparing the biological profile of a group of living systems with symptoms of the disease with the biological profile of a reference (or healthy) group of living systems, using a multivariate analysis, by measuring and integrating data from one or more metabolic, genetic and/or proteomic profiles;
(b) determining the impact of a series of samples of one or more synthetic compositions on the biological profile of the disease, in which samples the concentrations of the one or more synthetic compositions differ, using a multivariate analysis;
(c) preparing a set of multicomponent synthetic product mixtures which are expected to display a desired impact on the biological profile of the disease on the basis of the information obtained in step (b);
(d) determining the impact of the set of multicomponent mixtures as prepared in step (c) on the biological profile of the disease using multivariate analysis, so as to identify the said components and their respective concentrations.

2. Method according to claim 1, wherein after step (d) from the set of multicomponent synthetic product mixtures prepared in step (c) one or more mixtures are selected in a step (e), which selected mixtures display the desired impact on the biological profile of the disease.

3. Method according to claim 1 or 2, wherein in step (a) use is made of at least one spectrometric technique, at least one electromigration-based technique and/or at least one chromatographic technique to determine the profile of the disease.

4. Method according to any one of claims 1-3, wherein in step (b) use is made of at least one spectrometric technique, at least one electromigration-based technique and/or at least one chromatographic technique to determine the impact of the series of samples of the multicomponent mixture on the biological profile of the disease samples.

5. Method according to any one of claims 1-4 , wherein in step (d) use is made of at least one spectrometric technique, at least one electromigration-based technique and/or at least one chromatographic technique to determine the composition of the samples.

6. Method according to any one of claims 1-5, wherein use is made of two or more spectrometric techniques or electromigration-based techniques.

7. Method according to claim 6, wherein use is made of at least a nuclear magnetic resonance technique and a mass spectrometry technique or electromigration-based technique.

8. Method according to any one of claims 1-7, wherein the biological profile includes the metabolic, genetic and proteomic profiles.

9. Method according to any one of claims 1-8, wherein in step (a) the biological profiles are determined of at least one type of bodyfluid.

10. Method according to any one of claims 1-9, wherein in step (a) the biological profiles are determined of at least one type of tissue.

11. Method according to claim 10, wherein in step (a) the biological profiles are determined of at least two different types of bodyfluid.

12. Method according to any one of claims 1-11, wherein in step (a) the biological profiles are determined using one or more of the following biomarkers; genes, transcripts, proteins, metabolites and (trace) elements.

13. Method according to any one of claims 1-12, wherein the number of samples in step (b) is at least 2.

14. Method according to claim 13, wherein the number of samples in step (c) ranges from 5-100.

## Patentansprüche

1. In vitro-Verfahren zur Identifizierung von Bestandteilen und ihren entsprechenden Konzentrationen, die für Additive oder synergistische Wirkungen auf das biologische Profil einer Erkrankung innerhalb einer Gruppe von Lebensformen verantwortlich sind, in einer synthetischen Mehrfachkomponenten-Produktmischung, wobei die Mischung eine Mischung aus synthetischen Zusammensetzungen ist, die durch chemische Synthese hergestellt worden sind und nicht durch ein natürliches Verfahren, wobei das Verfahren die Schritte umfasst:
(a) Bestimmung eines biologischen Profils der Erkrankung durch Vergleichen des biologischen Profils einer Gruppe von Lebensformen mit Symptomen der Erkrankung mit dem biologischen Profil einer Referenz (oder gesunden)-Gruppe von Lebensformen, indem eine Multivarianzanalyse verwendet wird, durch Messen und Integrieren von Daten aus einem oder mehreren metabolischen, genetischen und/oder proteomischen Profil(en);
(b) Bestimmung der Auswirkungen einer Reihe von Proben einer oder mehreren synthetischen Zusammensetzungen auf das biologische Profil der Erkrankung, wobei die Konzentration der einen oder mehreren synthetischen Zusammensetzungen innerhalb der Proben verschieden sind, indem eine Multivarianzanalyse verwendet wird;
(c) Herstellung eines Satzes aus synthetischen Mehrfachkomponenten-Produktmischungen, von denen erwartet wird, dass sie eine gewünschte Auswirkung auf das biologische Profil der Erkrankung auf Grundlage der in Schritt (b) erhaltenen Information widerspiegeln;
(d) Bestimmung der Auswirkungen des Satzes aus Mehrfachkomponentenmischungen, die in Schritt (c) hergestellt worden sind, auf das biologische Profil der Erkrankung, indem eine Multivarianzanalyse verwendet wird, um die Bestandteile und ihre entsprechenden Konzentrationen festzustellen.

2. Verfahren gemäss Anspruch 1, wobei nach Schritt (d) des Satzes der synthetischen Mehrfachkomponenten-Produktmischung, die in Schritt (c) hergestellt wurde, eine oder mehrere Mischungen in Schritt (e) ausgewählt werden, wobei die ausgewählten Mischungen die gewünschten Auswirkungen auf das biologische Profil der Erkrankung widerspiegeln.

3. Verfahren gemäss Anspruch 1 oder 2, worin in Schritt
(a) von mindestens einer spektrometrischen Technik, mindestens einer auf Elektromigration basierenden Technik und/oder mindestens einer chromatografischen Technik Verwendung gemacht wird, um das Profil der Erkrankung zu bestimmen.

4. Verfahren gemäss irgendeinem der Ansprüche 1 bis 3, worin in Schritt (b) von mindestens einer spektrometrischen Technik, mindestens einer auf Elektromigration basierenden Technik und/oder mindestens einer chromatografischen Technik Verwendung gemacht wird, um die Auswirkungen der Reihe von Proben der Mehrfachkomponentenmischung auf das biologische Profil der Krankheitsproben zu bestimmen.

5. Verfahren gemäss irgendeinem der Ansprüche 1 bis 4, worin in Schritt (d) von mindestens einer spektrometrischen Technik, mindestens einer auf Elektromigration basierenden Technik und/oder mindestens einer chromatografischen Technik Verwendung gemacht wird, um die Zusammensetzung der Proben zu bestimmen.

6. Verfahren gemäss irgendeinem der Ansprüche 1 bis 5, wobei von zwei oder mehr spektrometrischen Techniken oder auf Elektromigration beruhenden Techniken Verwendung gemacht wird.

7. Verwendung gemäss Anspruch 6, wobei von mindestens einer auf Nuklearmagnetresonanztechnik und einer Massenspektrometrietechnik oder einer auf Elektromigration beruhenden Technik Verwendung gemacht wird.

8. Verfahren gemäss irgendeinem der Ansprüche 1 bis 7, wobei das biologische Profil metabolische, genetische und proteomische Profile einschliesst.

9. Verfahren gemäss irgendeinem der Ansprüche 1 bis 8, wobei in Schritt (a) die biologischen Profile von mindestens einer Art Körperflüssigkeit bestimmt werden.

10. Verfahren gemäss irgendeinem der Ansprüche 1 bis 9, wobei in Schritt (a) die biologischen Profile von mindestens einer Gewebeart bestimmt werden.

11. Verfahren gemäss Anspruch 10, wobei in Schritt (a) die biologischen Profile von mindestens zwei verschiedenen Arten von Körperflüssigkeiten bestimmt werden.

12. Verfahren gemäss irgendeinem der Ansprüche 1 bis 11, wobei in Schritt (a) die biologischen Profile unter Verwendung eines oder mehrerer der folgenden Biomarker bestimmt werden; Gene, Transkripte, Proteine, Metaboliten und (Spuren)-Elemente.

13. Verfahren gemäss irgendeinem der Ansprüche 1 bis 12, worin die Anzahl an Proben in Schritt (b) mindestens 2 beträgt.

14. Verfahren gemäss Anspruch 13, wobei die Anzahl an Proben in Schritt (c) von 5 bis 100 reicht.

## Revendications

1. Procédé in vitro pour identifier, dans un mélange de produits synthétiques à plusieurs composants, les composants et leurs concentrations respectives responsables des effets additifs ou synergiques sur le profil biologique d'une maladie dans un groupe de systèmes vivants, lequel mélange est un mélange de compositions synthétiques qui ont été produites par synthèse chimique, non par un processus naturel, lequel procédé comprend les étapes consistant à :
(a) déterminer un profil biologique de la maladie par comparaison du profil biologique d'un groupe de systèmes vivants présentant les symptômes de la maladie avec le profil biologique d'un groupe de références (ou sain) de systèmes vivants, au moyen d'une analyse multivariée, par mesure et intégration des données provenant d'un ou plusieurs profils métaboliques, génétiques et/ou protéomiques ;
(b) déterminer l'impact d'une série d'échantillons d'une ou plusieurs compositions synthétiques sur le profil biologique de la maladie, dans lesquels échantillons les concentrations de la une ou plusieurs compositions synthétiques diffèrent, au moyen d'une analyse multivariée ;
(c) préparer un ensemble de mélanges à base de produits synthétiques à plusieurs composants dont on s'attend à ce qu'ils présentent un impact souhaité sur le profil biologique de la maladie sur la base des informations obtenues dans l'étape (b) ;
(d) déterminer l'impact de l'ensemble de mélanges à plusieurs composants tel que préparé dans l'étape (c) sur le profil biologique de la maladie au moyen d'une analyse multivariée, de manière à identifier lesdits composants et leurs concentrations respectives.

2. Procédé selon la revendication 1, dans lequel après l'étape (d), à partir de l'ensemble de mélanges à base de produits synthétiques à plusieurs composants séparé dans l'étape (c), un ou plusieurs mélanges sont choisis dans une étape (e), lesquels mélanges choisis présentent l'impact souhaité sur le profil biologique de la maladie.

3. Procédé selon la revendication 1 ou 2, dans lequel dans l'étape (a), au moins une technique spectrométrique, au moins une technique à base d'électromigration et au moins une technique chromatographique sont utilisées afin de déterminer le profil de la maladie.

4. Procédé selon l'une quelconque des revendications 1 à 3, dans lequel dans l'étape (b), au moins une technique spectrométrique, au moins une technique à base d'électromigration et/ou au moins une technique chromatographique sont utilisées afin de déterminer l'impact des séries d'échantillons du mélange à plusieurs composants sur le profil biologique des échantillons de la maladie.

5. Procédé selon l'une quelconque des revendications 1 à 4, dans lequel dans l'étape (d), au moins une technique spectrométrique, au moins une technique à base d'électromigration et/ou au moins une technique chromatographique sont utilisées afin de déterminer la composition des échantillons.

6. Procédé selon l'une quelconque des revendications 1 à 5, dans lequel deux ou plusieurs techniques spectrométriques ou techniques à base d'électromigration sont utilisées.

7. Procédé selon la revendication 6, dans lequel au moins une technique de résonance magnétique nucléaire et une technique de spectrométrie de masse ou une technique à base d'électromigration sont utilisées.

8. Procédé selon l'une quelconque des revendications 1 à 7, dans lequel le profil biologique comporte les profils métaboliques, génétiques et protéomiques.

9. Procédé selon l'une quelconque des revendications 1 à 8, dans lequel dans l'étape (a), les profils biologiques d'au moins un type de liquide organique sont déterminés.

10. Procédé selon l'une quelconque des revendications 1 à 9, dans lequel dans l'étape (a), les profils biologiques d'au moins un type de tissu sont déterminés.

11. Procédé selon la revendication 10, dans lequel dans l'étape (a), les profils biologiques d'au moins deux types différents de liquide organique sont déterminés.

12. Procédé selon l'une quelconque des revendications 1 à 11, dans lequel dans l'étape (a) les profils biologiques sont déterminés au moyen d'un ou plusieurs des biomarqueurs suivants : gènes, transcrits, protéines, métabolites et éléments (trace).

13. Procédé selon l'une quelconque des revendications 1 à 12, dans lequel le nombre d'échantillons dans l'étape (b) est au moins 2.

14. Procédé selon la revendication 13, dans lequel le nombre d'échantillons dans l'étape (c) varie de 5 à 100.
